(19) Europäisches Patentamt
European Patent Office
Office européen des brevets

(11) EP 3 983 789 B1

(12) EUROPEAN PATENT SPECIFICATION

(45) Date of publication and mention of the grant of the patent:
**31.07.2024 Bulletin 2024/31**

(21) Application number: **20737543.7**

(22) Date of filing: **12.06.2020**

(51) International Patent Classification (IPC):
***G01N 27/48*** *(2006.01)* ***G01N 33/487*** *(2006.01)*
***G01N 30/00*** *(2006.01)*

(52) Cooperative Patent Classification (CPC):
**G01N 27/48; G01N 33/48714**

(86) International application number:
**PCT/IB2020/055545**

(87) International publication number:
**WO 2020/250200 (17.12.2020 Gazette 2020/51)**

(54) **METHOD FOR DETERMINING THE CONCENTRATION OF 4-[(4-METHYL-1-PIPERAZINYL)METHYL]-N-(4-METHYL-3-{[4-(3-PYRIDINYL)-2- PYRIMIDINYL] - AMINO} PHENYL)BENZ AMIDE (IMATINIB) IN PLASMA SAMPLES**

VERFAHREN ZUR BESTIMMUNG DER KONZENTRATION VON 4-[(4-METHYL-1-PIPERAZINYL)METHYL]-N-(4-METHYL-3-{[4-(3-PYRIDINYL)-2-PYRIMIDINYL]-AMINO}PHENYL)BENZAMID (IMATINIB) IN PLASMAPROBEN

PROCÉDÉ DE DÉTERMINATION DE LA CONCENTRATION EN 4-[(4-MÉTHYL-1-PIPÉRAZINYL)MÉTHYL]-N-(4-MÉTHYL-3-{[4-(3-PYRIDINYL)-2-PYRIMIDINYL]-AMINO}PHÉNYL)BENZAMIDE (IMATINIB) DANS DES ÉCHANTILLONS DE PLASMA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.06.2019 IT 201900008808**

(43) Date of publication of application:
**20.04.2022 Bulletin 2022/16**

(73) Proprietors:
- **UNIVERSITA' CA' FOSCARI**
  **30123 Venezia (IT)**
- **Centro Di Riferimento Oncologico**
  **33081 Aviano (IT)**

(72) Inventors:
- **BONAZZA, Gregorio**
  **30135 Venezia (IT)**
- **DANIELE, Salvatore**
  **35137 Padova (IT)**
- **POLO, Federico**
  **31030 Castelcucco (IT)**
- **TOFFOLI, Giuseppe**
  **33077 Sacile (IT)**
- **TARTAGGIA, Stefano**
  **31039 Riese Pio X (IT)**

(74) Representative: **Palladino, Saverio Massimo et al**
**Notarbartolo & Gervasi S.p.A.**
**Viale Achille Papa, 30**
**20149 Milano (IT)**

(56) References cited:
**WO-A1-00/67011 US-A1- 2018 136 161**

- **E. HAMMAM: "Voltammetric behavior and quantification of the anti-leukemia drug imatinib in bulk form, pharmaceutical formulation, and human serum at a mercury electrode", CANADIAN JOURNAL OF CHEMISTRY, vol. 82, no. 7, 1 January 2004 (2004-01-01), pages 1203 - 1209, XP055662178**
- **RODRIGUEZ J ET AL: "Voltammetric determination of Imatinib (Gleevec) and its main metabolite using square-wave and adsorptive stripping square-wave techniques in urine samples", TALANTA, ELSEVIER, AMSTERDAM, NL, vol. 66, no. 1, 31 March 2005 (2005-03-31), pages 202 - 209, XP027860161, ISSN: 0039-9140, [retrieved on 20050331]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- JUANA RODRÍGUEZ ET AL: "Electrochemical sensor for leukemia drug imatinib determination in urine by adsorptive striping square wave voltammetry using modified screen-printed electrodes", ELECTROCHIMICA ACTA, vol. 269, 1 April 2018 (2018-04-01), AMSTERDAM, NL, pages 668 - 675, XP055663378, ISSN: 0013-4686, DOI: 10.1016/j.electacta.2018.03.051
- EVA KRALJ ET AL: "Simultaneous measurement of imatinib, nilotinib and dasatinib in dried blood spot by ultra high performance liquid chromatography tandem mass spectrometry", JOURNAL OF CHROMATOGRAPHY B: BIOMEDICAL SCIENCES & APPLICATIONS., vol. 903, 1 August 2012 (2012-08-01), NL, pages 150 - 156, XP055663385, ISSN: 1570-0232, DOI: 10.1016/j.jchromb.2012.07.011
- DAVIES A ET AL: "Simultaneous determination of nilotinib, imatinib and its main metabolite (CGP-74588) in human plasma by ultra-violet high performance liquid chromatography", LEUKEMIA RESEARCH, NEW YORK,NY, US, vol. 34, no. 6, 1 June 2010 (2010-06-01), pages 702 - 707, XP027015257, ISSN: 0145-2126, [retrieved on 20091209]

## Description

### FIELD OF THE INVENTION

The present invention relates to a method for determining the concentration of 4-[(4-methyl-1-piperazinyl)methyl] -N-(4-methyl-3-{[4-(3-pyridinyl)-2-pyrimidinyl]-amino}phenyl)benzamide in plasma samples, which comprises the extraction of the compound from the sample and the subsequent measurement of the concentration thereof electrochemically.

### STATE OF THE ART

The compound 4-[(4-methyl-1-piperazinyl)methyl]-N-(4-methyl-3-{[4-(3-pyridinyl)-2-pyrimidinyl]-amino}phenyl)benzamide, having the formula reported below, is known in the medical-pharmaceutical field with the INN name Imatinib, which will be used in the rest of this text.

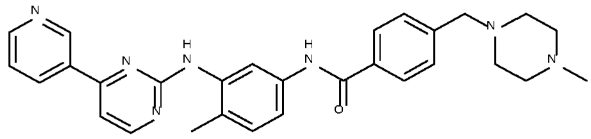

Imatinib

Imatinib is an anti-leukemic drug used primarily for the first-line treatment of certain forms of cancers, including the acute lymphoblastic leukemia (ALL), certain types of gastrointestinal stromal tumors (GIST) and, in particular, the Philadelphia chromosome-positive chronic myeloid leukemia (CML). Imatinib acts by inhibiting the aberrant enzyme Bcr-Abl tyrosine kinase, a specific chromosomal anomaly.

The compound is mainly metabolised by the enzymes CYP3A4 and CYP3A5 (cytochromes P450 3A4 and P450 3A4, respectively) to give mainly the active metabolite, N-des-methyl Imatinib (des-Imatinib, i.e. Imatinib from which the terminal methyl on the piperazine ring is removed), whose maximum concentration in plasma is usually not higher than about 15% compared to that of Imatinib itself.

Imatinib has demonstrated remarkable therapeutic efficacy with positive and long-lasting clinical responses, which have led to a prolonged survival of the patients. However, the pharmacological treatment is not free of toxicity, and produces side effects such as oedema, nausea, vomiting and diarrhea.

Imatinib has a narrow therapeutic window, which means that the concentrations within which pharmacotherapy is effective or toxic are quite close. Current clinical protocols for the treatment of CML suggest keeping Imatinib concentration in the bloodstream within 1000 and 3000 ng/mL (2-6 $\mu$M). Most patients are treated with a 400 mg/day dose, which is eventually adjusted based on the clinical manifestations after drug administration.

Patent application CN104804079 A describes a specific antibody for Imatinib to be used for detecting the molecule, but the method gives rise to a qualitative response (presence or absence of Imatinib) which does not allow to obtain a reliable quantitative datum with regard to maintaining the concentration in the blood of the drug within the limits indicated above.

Among the therapies that use the compound, there are important customized pharmacological treatments adapted to avoid both excessive and insufficient dosages, thus allowing a more effective chemotherapeutic use of the compound.

In this regard, therapeutic drug monitoring (technique known in the medical sector as TDM) has become essential to help in determining the optimal drug dosage for the single patient. For this purpose, it is necessary to be able to determine with sufficient accuracy the concentration levels of Imatinib or the metabolites thereof in the patient's blood at fixed times after administration, in order to be able to correlate these concentration levels with the reactions of the single patient.

The method is illustrated for example in patent application EP2251042 A2, which describes a method for optimizing an Imatinib-based therapy for the specific patient which comprises the steps of: administering a predetermined fixed quantity of Imatinib (or a pharmaceutically acceptable salt thereof) to a patient; collecting at least one blood sample from the patient; determining the minimum plasma level ($C_{min}$) reached by Imatinib between two successive administrations as well as the rates that allow the Bcr-Abl transcripts to be reduced by at least a factor 1000; identifying an optimal threshold value of $C_{min}$ which allows to obtain the desired result in terms of reduction of Bcr-Abl transcripts; and adjusting the dosage of Imatinib applied to the single patient. In this application the Imatinib values in plasma, in particular the $C_{min}$ values, are determined by HPLC coupled to tandem mass spectrometry with electrospray ionization.

Similarly, patent application CN104483407 A concerns the determination of the concentration of Imatinib in

biological samples by HPLC coupled to tandem mass spectrometry.

The methods and instrumentations described in the last two documents allow the precise determination of the concentration of the metabolites of Imatinib in biological samples, but are poorly suitable for use in a TDM methodology due above all to the poor portability of the instruments, the relatively long analysis times, and the need for centralised analysis laboratories and qualified personnel. An effective use of the TDM methodology requires instead simple and fast analytical protocols for the detection and control of the concentration of the drug in the patient's blood.

Methods for detection of Imatinib in human serum or urine by adsorptive stripping voltammetry are described in Hammam E. et al (Voltammetric behavior and quantification of the anti-leukemia drug imatinib in bulk form, pharmaceutical formulation, and human serum at a mercury electrode, February 2011, Canadian Journal of Chemistry 82(7):1203-1209), Rodriguez J. et al (Voltammetric determination of Imatinib (Gleevec) and its main metabolite using square-wave and adsorptive stripping square-wave techniques in urine samples, April 2005, Talanta 66(1):202-9) and Rodriguez et al (Electrochemical sensor for leukemia drug imatinib determination in urine by adsorptive striping square wave voltammetry using modified screen-printed electrodes, March 2018, Electrochimica Acta 269(4)).

The need to have an analytical methodology available for the determination of Imatinib in biological samples that is completely compatible with the requirements of the TDM technique is therefore still felt in the field.

The object of the present invention is to provide an analytical method for the fast and accurate determination of Imatinib in a blood plasma sample.

## SUMMARY OF THE INVENTION

This object is achieved with the present invention, which relates to a method for detecting Imatinib in a plasma sample, which comprises the following steps:

a) loading the plasma sample onto a simplified liquid extraction column supported on a solid phase;
b) loading the column with a basic aqueous solution;
c) eluting Imatinib from the simplified liquid extraction column supported on a solid phase using an organic solvent chosen among tetrahydrofuran, methyl-ter-butyl ether, ethyl ether, acetonitrile, ethyl acetate, butyl acetate and mixtures thereof, collecting at the bottom of the column a solution containing Imatinib;
d) adding to the Imatinib solution obtained in step c) tetrabutylammonium hexafluorophosphate at a concentration between 1 and 100 mM and adding to the same solution a phosphoric acid concentrated solution, glacial acetic acid and ultrapure water;
e) analyzing the mixture obtained in step d) by adsorptive stripping voltammetry, whose waveform (potential, E, as a function of time, t) is constituted by a first accumulation period in which the potential is kept constant at a first value, a second period of linear decrease of the potential up to a second value, and a third period of linear increase of the potential up to returning to the first value, in which said first and second values of potential are respectively +1.1 V and +0.3 V with respect to a reference electrode.

## BRIEF DESCRIPTION OF THE DRAWINGS

The invention will be described in the following with reference to the figures, in which:

- Fig. 1 schematically shows the phases of the separation of Imatinib, by means of a simplified liquid extraction column supported on a solid phase, from the other plasma components and from possible interfering compounds in the subsequent voltammetric analysis;
- Fig. 2 shows a possible waveform used in the measurement of adsorbitive stripping voltammetry;
- Fig. 3 shows two curves relating to voltammetric measurements of solutions containing Imatinib (continuous lines) and, for comparison, two similar curves on solutions having the same composition (solvent and additives) but not containing Imatinib (dashed curves);
- Fig. 4 schematically shows an instrument for carrying out the Imatinib analysis in an automated mode;
- Fig. 5 shows the voltammetric signals recorded in a series of plasma extracts added with increasing concentrations of Imatinib;
- Fig. 6 shows the linear regression line of the data obtained from the curves of Fig. 5, in particular the data of the peak currents as a function of concentration.

## DETAILED DESCRIPTION OF THE INVENTION

In the present invention, the following terms and abbreviations have the meanings specified herein:

- unless otherwise indicated, all percentages and concentrations in the following description are molar (M), millimolar (mM), micromolar (μM) or nanomolar (nM);
- "analyte" indicates a substance whose presence or quantity (or concentration) must be sought in an analysis; in the present invention, the analyte is the compound Imatinib;
- "LOD", i.e. "limit of detection", indicates the minimum amount of analyte that gives rise to a detection (that is, that provides an indication different from "absence of the sought analyte");
- "LOQ", i.e. "limit of quantification", indicates the minimum amount of analyte which gives rise to a reliable quantitative measurement of the quantity or concentration thereof; this value is always higher than the LOD;
- the adsorptive stripping voltammetry technique will be indicated with the abbreviation "AdSV" commonly used in the field;
- the simplified liquid extraction columns supported on a solid phase will be indicated with the acronym "SLE" commonly used in the field.

In the present text and in the claims, the term "phosphoric acid concentrated solution" means an aqueous solution of orthophosphoric acid, $H_3PO_4$, at a concentration equal to 85% by weight.

In the present text and in the claims, the term "ultrapure water" means type I water according to the ASTM D1193-91 standard, having a conductivity equal to or less than $5.5 \times 10^{-6}$ S/m; water of this quality can be produced in the laboratory from mains water through a Milli-Q® purifier of the company Millipore Corp., and type I ultrapure water is often referred to for simplicity in the analytical field as Milli-Q water.

The method of the invention comprises two main phases: a first phase of selective extraction of Imatinib from the initial biological sample and a subsequent phase of electrochemical analysis of the Imatinib solution thus obtained.

In step a) of the method (Fig. 1.a), a plasma sample taken from a patient to whom Imatinib had been administered is loaded into an SLE column and forced into the column using a flow of inert gas or by applying vacuum downstream the column itself; in the figure, the full triangles represent the Imatinib molecules, the stars the interferents in the analysis.

The extraction technique used is a variant of liquid-liquid extraction, common in chemistry, in which however the extraction liquid is distributed on the surface and/or in the pores of a solid support phase, generally a zeolite or silica. SLE columns are widely available on the market; columns suitable for the purposes of the present invention are for example NOVUM columns sold in Italy by the company Phenomenex srl from Castel Maggiore (BO).

One of the advantages of the method of the invention is to be able to work with minimal quantities of plasma, so as to allow limited blood sampling in already debilitated patients, or the use also in other parallel analyses of the taken sample. Typically, the volume of plasma used in the method of the invention is about 20-200 μL.

In step b), a basic aqueous solution is added to the column already loaded with plasma (Fig. 1.b). This step is necessary because Imatinib at physiological pH is in balance with its protonated form, and the addition of the base allows to neutralize the acidity of the drug and transform it into its neutral form, suitable for subsequent extraction by means of the organic solvent. The solution used in this step is an aqueous solution of a base preferably chosen among sodium hydroxide, potassium hydroxide, potassium phosphate, sodium phosphate, sodium carbonate, potassium carbonate, sodium borate and, even more preferably, an aqueous solution of ammonia; if a solution of ammonia is used, the pH of the resulting Imatinib solution is about 10.5.

In step c), an organic solvent which constitutes the eluent of the method is passed in the SLE column under nitrogen flow or by applying a vacuum downstream the same (Fig. 1.c); the preferred eluent for the purposes of the present invention is ethyl acetate. The eluent carries the neutral Imatinib, while the possible interferents in the subsequent voltammetric analysis remain in the column. Preferably, the elution with ethyl acetate is accomplished by adding consecutive aliquots (from two to ten) of the solvent. Downstream the column an Imatinib solution in said solvent is obtained. Typically, the volume of eluent used (in a single elution step or divided into aliquots used in subsequent elution steps) is between about 10 and 25 times, preferably about 20 times, the initial volume of plasma.

In step d), tetrabutylammonium hexafluorophosphate ($(CH_3CH_2CH_2CH_2)_4NPF_6$, indicated in the rest of the text with the abbreviation $TBAPF_6$) is added to said solution at a concentration between 1 and 100 mM; afterwards phosphoric acid and acetic acid are added in quantities dependent on the volume of eluate collected from the column in step c), respecting the following proportions: for 100 parts by volume of eluate, between 3.5 and 12 parts by volume of a phosphoric acid concentrated solution, between 4.5 and 12 parts by volume of glacial acetic acid, and between 1 and 6 parts by volume of water are added. The acids are added to obtain better defined and more intense voltammetric signals.

Finally, in step e), the solution thus obtained is analysed by means of the AdSV technique.

The measure is carried out in a three electrodes electrochemical cell, comprising a working electrode, a reference electrode and a counter electrode. As a working electrode it is possible to use a glassy carbon electrode (known in the sector as GCE), other carbon-based materials (such as graphene and boron-doped diamond), or a platinum electrode; a silver wire is used as reference electrode, used instead of the common Ag/AgCl reference electrode (saturated with KCl) to avoid contamination of the sample with chlorine ions; and a platinum spiral as a counter electrode.

Fig. 2 shows a possible profile of the potential in a measure cycle: the potential is kept constant at the value +1.1 (with respect to the reference electrode) for an accumulation time, exemplified in the figure as 30 s; the potential is then brought linearly to the value of +0.3 with respect to the reference electrode, in a time exemplified in the figure as 12 s, and immediately returned linearly to the initial value in a time exemplified in the figure as a further 12 s.

The inventors have verified that by performing a scan of potential (between +0.3 and +1.1 V) in the positive region with respect to the reference electrode, three peaks are detected, identified with the numbers 1, 2 and 3 in Fig. 3, all attributable uniquely to Imatinib; the dotted line corresponds to the voltammogram recorded in the absence of Imatinib. The inventors have also observed that the charges associated with the peaks 2 and 3, or even the peak currents, depend linearly on the concentration of the analyte, at least in the concentration range between 0.05 and 0.9 μM, thus allowing the quantitative analysis of the sample. In fact, the inventors have verified through various dedicated tests that in the concentration range indicated above the measured charge is linked to the concentration of the analyte with a relationship of the type:

$$Q_m = P \times C + K$$

wherein $Q_m$ is the measured charge associated with the stripping peak (measured in nanoCoulomb, nC), P is the slope of the straight line obtained from the linear regression of the experimental data, C the concentration (nM) and K the value of the intercept of the straight line of calibration. The determination coefficient $R^2$ measured in various tests was always higher than 0.98, confirming an excellent linearity of the charge measurement response as a function of the analyte concentration.

With these tests, the following analytical performance data were also obtained:

- dynamic range of the method: $5 \times 10^{-8} \div 1 \times 10^{-6}$ M;
- LOD: $1.1 \times 10^{-8}$ M;
- LOQ: $3.7 \times 10^{-8}$ M.

These values refer to the plasma sample diluted by addition of ethyl acetate for the extraction; the dynamic range of the method becomes $1 \times 10^{-6} \div 2 \times 10^{-5}$ M referred to the original sample; also with regards to the LOD and LOQ values the plasma dilution factor must be duly taken into account.

An automated instrument 1 that can be used for the realisation of the above described analysis method is schematically represented in Fig. 4. The main elements of the instrument 1 are the simplified liquid extraction column supported on a solid phase 10, an analyser 11 (which can be a voltammeter or a potentiostat) equipped with a three-electrode measuring head 12, and a microprocessor 13 (which could also be a personal computer) that controls the execution of the different steps of the method, receives the data from the analyser 11, processes them and generates the results of the analysis.

The other parts of the instrument are:

- a sample introduction chamber 14 (defined below as injection chamber) connected downstream, through a two-way valve $V_1$, to the inlet opening of the column 10;
- a tank 15 for a basic aqueous solution, connected through a two-way valve $V_2$ to the inlet opening of the column 10;
- a tank 16 for an eluent, connected through a two-way valve $V_3$ to the inlet opening of the column 10;
- a container 17 for the collection of the Imatinib sample extracted from the column 10 through the eluent; the container 17 is fixed to handling means 18, which at the end of the elution and therefore of the collection of the sample in the container, move it under the measuring head 12;
- a tank 19 for a solution of tetrabutylammonium hexafluorophosphate, connected through a two-way valve $V_7$ to the line for the addition of the reagent to the solution in container 17;
- a tank 20 for a phosphoric acid concentrated solution and glacial acetic acid in ultrapure water, connected through a two-way valve $V_8$ to the line for the addition of said solution to the solution in container 17;
- the entry of the sample into the column 10, as mentioned above, can be favoured by an overpressure of an inert gas upstream injection chamber 14 or by a negative pressure downstream column 10. These two possibilities correspond to two alternative examples of the instrument, both illustrated in Fig. 4 through dotted lines. In the first example, the injection chamber 14 is connected upstream through a two-way valve $V_4$ to a source 21 of a pressurized gas; in the alternative example, the column 10 is connected downstream through a three-way valve $V_5$ to a suction pump 21'. To increase the versatility of the instrument, this can also include both the source 21 of pressurized gas and the suction pump 21';
- the valves $V_1$, $V_2$, $V_3$, $V_4$, $V_5$, $V_7$ and $V_8$ are connected to the microprocessor 13 respectively through power and data lines $L_1$, $L_2$, $L_3$, $L_4$, $L_5$, $L_7$ and $L_8$ (the lines $L_4$ and $L_5$, as well as the valves $V_4$ e $V_5$, can be both present in the

system or be present in alternative to each other); at least the valves $V_7$ and $V_8$ are of the volumetric type, i.e. capable of dosing a desired quantity of a fluid; moreover, power lines and data transfer lines (represented cumulatively in the figure by line $L_6$) connect the microprocessor 13 to the analyser 11.

For the execution of the method of the invention, after the injection of the sample to be analysed into the chamber 14, the operator can start the automated analysis with a command to the microprocessor 13. Following this command, the microprocessor opens the valve Vi; simultaneosly, the valve $V_4$, is opened, or the suction pump 21' is activated and the three-way valve $V_5$ is opened in a position such that the pump is put in a fluid connection with the column 10. When (through the source of pressurized gas 21 or the pump 21') the sample has been completely introduced into the column 10, the valve $V_4$ is closed or, alternatively, the valve $V_5$ is opened in the direction of the discharge of liquids and the pump 21' is isolated from the system. Subsequently, the microprocessor 13 commands the closure of the valve $V_1$ and the opening of the valve $V_2$ letting the basic aqueous solution from tank 15 enter the column 10. Up to this step, any liquids leaving the column 10 are sent to a discharge line (not shown in the figure) and discharged outside the system. In the subsequent step, the microprocessor 13 moves, through the movement of the means 18, the container 17 under the outlet opening of the column 10 and commands the closure of valve $V_2$ and the opening of valve $V_3$ letting the eluent present in tank 16 enter into column 10; in this way the analyte is transported by the eluent to the container 17. Subsequently, the microprocessor 13 commands the opening of the valves $V_7$ and $V_8$, adding the solution of tetrabutylammonium hexafluorophosphate and the phosphoric acid concentrated solution and glacial acetic acid in ultrapure water to the solution in the container 17. In the subsequent step the microprocessor, through the means 18, moves the container 17 under the analyser 11, and through the line $L_6$ commands the vertical movement of the head 12 and the consequent immersion of the electrodes into the solution to be analysed; in the figure, the dotted lines show the container 17 and the head 12 in the measuring position. In an alternative example not shown in the figure, the container 17, once positioned under the head 12, might rise so as to immerse the electrodes into the solution to be analysed. Finally, the measurement is performed and through the line $L_6$ the microprocessor 13 receives and processes the data of the analyser 11 generating the result of the analysis. At the end of the analysis of a sample, the head 12 is extracted from the container 17, the microprocessor (through the means 18) moves a container 17' under said head and causes the electrodes to immerse into the liquid contained in the latter container; the liquid in container 17' is a washing liquid, which eliminates the traces of the sample just analysed from the surface of the electrodes, to prepare them for a new measurement. The washing liquid in the container 17' is preferably refreshed after each cleaning of the electrodes, in order to avoid the accumulation of residues of previous analyses which might lead to cross-contaminations in an analysis sequence. The washing liquid can come from the tank 16 or from a dedicated tank (neither of the two options is shown in the Figure).

The invention will be further described by the following experimental part.

Instrumentation

Plasma separations to obtain Imatinib solutions in ethyl acetate were carried out with Novum columns of Phenomenex® Inc.

A CHI 920 bi-potentiostat of the company CH Instruments in Austin, Texas, USA, was used for the voltammetric measurements.

The working electrode used in the voltammetric analyses was a glassy carbon disc (GCE) (Ø = 3 mm), mechanically polished with diamond suspensions (particle diameter 0.1 $\mu$m) placed over a Buehler microtextile, and then washed with distilled water and ethyl acetate.

**EXAMPLE 1**

To confirm the validity of the method for the quantitative analysis, the inventors have prepared a series of synthetic samples of Imatinib solution in ethyl acetate with a concentration between $5 \times 10^{-8}$ and $9 \times 10^{-7}$ M, which were measured with the AdSV technique described above and using the charge associated with peak 3 in Fig. 3. The ethyl acetate solution also contained the other reagents specified above in step d). From the tests of the type reported in Fig. 5, the following linear regression equation is obtained (Fig. 6):

$$Q = 0.846C + 11$$

wherein Q is the charge measured in nC and C the concentration nM, with a determination coefficient $R^2$ equal to 0.991.

## EXAMPLE 2

Blood was taken from three patients with chronic myeloid leukemia, subjected to chemotherapy with Imatinib, 24 hours after administration of the drug. The following procedure was performed for each of the samples analysed.
The plasma was separated from the blood samples by centrifugation.
An SLE column was loaded with 50 μL of plasma and subsequently with 50 μL of an aqueous solution containing 0.087 mol/L of ammonia; said ammonia solution was used to obtain the whole drug in its deprotonated form. The sample and the solution were pushed into the solid phase of the column by means of a nitrogen flow (purity 99.99%, supplied by SIAD).
The Imatinib present in the column was eluted using two aliquots of 500 μL each of ethyl acetate, forced to pass through the column by a flow of pure nitrogen. The final volume of the eluate (ethyl acetate containing Imatinib) was 850 ± 10 μL, because part of the solvent remains in the solid phase of the column.
A solution containing 70 μL of phosphoric acid, 55 μL of acetic acid, 25 μL of water was added to the eluate. $TBAPF_6$ was then added in such a quantity to obtain a final solution of 0.025 mM concentration, starting from a saturated solution of the salt in acetonitrile at room temperature.
AdSV measurements for detecting Imatinib were performed in the same three-electrode cell of Example 1. A control test was carried out on each of the three plasma samples using the validated method, and currently used in the field (HPLC-MS test), to verify the accuracy of the results obtained with the method of the invention.
To obtain a statistically significant result, each test was repeated at least three times.
The test results are summarised in the following table:

| Sample | $C_{Ref}$(μM) (DRS%) | $C_I$(μM) (DRS%) | ER% |
|---|---|---|---|
| Patient 1 | 4.64 (≤ 10) | 4.73 (4) | 1.9 % |
| Patient 2 | 2.20 (≤ 10) | 2.04 (6) | 6.4 % |
| Patient 3 | 1.29 (≤ 10) | 1.69 (6) | 31 % |

The table shows:

- in the second column the analyte concentration data ($C_{Ref}$) in the sample obtained with the reference method, and in brackets the relative percentage standard deviation value (DSR%) of the measurement;
- in the third column the analyte concentration data ($C_I$), and in brackets the DSR% data, obtained on the same plasma samples with the method of the invention;
- in the last column the relative error (ER) of the method of the invention with respect to CRef.

### Claims

**1.** Method for the detection of Imatinib in a blood plasma sample, comprising the following steps:

a) loading the plasma sample onto a simplified liquid extraction column supported on a solid phase;
b) loading the column with a basic aqueous solution;
c) eluting Imatinib from the simplified liquid extraction column supported on a solid phase using an organic solvent chosen among tetrahydrofuran, methyl-ter-butyl ether, ethyl ether, acetonitrile, ethyl acetate, butyl acetate and mixtures thereof, collecting at the bottom of the column a solution containing Imatinib;
d) adding to the Imatinib solution obtained in step c) tetrabutylammonium hexafluorophosphate at a concentration between 1 and 100 mM and adding to the same solution a phosphoric acid concentrated solution, glacial acetic acid and ultrapure water;
e) analyzing the mixture obtained in step d) by adsorptive stripping voltammetry, whose waveform (potential, E, as a function of time, t) is constituted by a first accumulation period in which the potential is kept constant at a first value, a second period of linear decrease of the potential up to a second value, and a third period of linear increase of the potential up to returning to the first value, in which said first and second values of potential are respectively +1.1 V and +0.3 V with respect to a reference electrode.

**2.** Method according to claim 1, in which in step a) a plasma volume between 20 and 200 μL is used.

3. Method according to one of the claims 1 or 2, in which step a) is carried out using a flow of inert gas or by applying a vacuum downstream said column.

4. Method according to any one of the preceding claims, in which the basic solution used in step b) is an aqueous solution of a base chosen among sodium hydroxide, potassium hydroxide, potassium phosphate, sodium phosphate, sodium carbonate, potassium carbonate, sodium borate and ammonia.

5. Method according to any one of the preceding claims, in which in step b) an aqueous solution of ammonia is used and the pH of the sample is brought to a value of about 10.5.

6. Method according to any one of the preceding claims, in which step b) is carried out using a flow of inert gas or by applying a vacuum downstream said column.

7. Method according to any one of the preceding claims, in which the solvent used as eluent in step c) is ethyl acetate.

8. Method according to any one of the preceding claims, in which step c) is carried out using a flow of inert gas or by applying a vacuum downstream said column.

9. Method according to any one of the preceding claims, in which step c) is carried out by adding said solvent in two to ten consecutive portions.

10. Method according to any one of the preceding claims, where the volume of solvent used in step c) is between 10 and 25 times the initial volume of plasma.

11. Method according to any one of the preceding claims, in which the measure of step e) is carried out in an electrochemical cell comprising a working electrode, a reference electrode and a counter electrode.

12. Method according to claim 11, in which a glass carbon electrode or a platinum electrode is used as a working electrode, a silver wire is used as a reference electrode, and a platinum spiral is used as a counterelectrode.

**Patentansprüche**

1. Verfahren zum Nachweis von Imatinib in einer Blutplasmaprobe, das die folgenden Schritte umfasst:

   a) Beschicken einer vereinfachten, festphasenunterstützten Flüssigextraktionssäule mit einer Plasmaprobe;
   b) Beschicken der Säule mit einer basischen wässrigen Lösung;
   c) Eluieren von Imatinib aus der vereinfachten, festphasenunterstützten Flüssigextraktionssäule unter Verwendung eines organischen Lösungsmittels, das aus Tretrahydrofuran, Methyl-tert-butylether, Ethylether, Acetonitril, Ethylacetat, Butylacetat und Gemischen davon ausgewählt ist, wodurch am Boden der Säule eine Imatinib enthaltende Lösung gesammelt wird;
   d) Zugeben von Tetrabutylammoniumhexafluorphosphat in einer Konzentration zwischen 1 und 100 mM zu der in Schritt c) erhaltenen Imatinib-Lösung und Zugeben einer konzentrierten Phosphorsäurelösung, Eisessig und Reinstwasser zu derselben Lösung;
   e) Analysieren des in Schritt d) erhaltenen Gemischs durch Adsorptions-Stripping-Voltammetrie, deren Kurvenform (Potenzial E als Funktion der Zeit t) aus einem ersten Ansammlungszeitraum, in dem das Potenzial konstant auf einem ersten Wert gehalten wird, einem zweiten Zeitraum linearer Senkung des Potenzials bis zu einem zweiten Wert und einem dritten Zeitraum linearer Erhöhung des Potenzials bis zur Rückkehr zum ersten Wert besteht, wobei der erste und der zweite Wert des Potenzials +1,1 V bzw. +0,3 V in Bezug auf eine Referenzelektrode sind.

2. Verfahren nach Anspruch 1, wobei in Schritt a) ein Plasmavolumen zwischen 20 und 200 $\mu$l verwendet wird.

3. Verfahren nach einem der Ansprüche 1 oder 2, wobei Schritt a) unter Verwendung eines Stroms aus Inertgas oder durch Anwenden eines Vakuums stromabwärts der Säule durchgeführt wird.

4. Verfahren nach einem der vorhergehenden Ansprüche, wobei die in Schritt b) verwendete basische Lösung eine wässrige Lösung einer Base ist, die aus Natriumhydroxid, Kaliumhydroxid, Kaliumphosphat, Natriumphosphat, Natriumcarbonat, Kaliumcarbonat, Natriumborat und Ammoniak ausgewählt ist.

5. Verfahren nach einem der vorhergehenden Ansprüche, wobei in Schritt b) eine wässrige Lösung aus Ammoniak verwendet wird und der pH der Probe auf einen Wert von etwa 10,5 gebracht wird.

6. Verfahren nach einem der vorhergehenden Ansprüche, wobei Schritt b) unter Verwendung eines Stroms aus Inertgas oder durch Anwenden eines Vakuums stromabwärts der Säule durchgeführt wird.

7. Verfahren nach einem der vorhergehenden Ansprüche, wobei das in Schritt c) als Eluent verwendete Lösungsmittel Ethylacetat ist.

8. Verfahren nach einem der vorhergehenden Ansprüche, wobei Schritt c) unter Verwendung eines Stroms aus Inertgas oder durch Anwenden eines Vakuums stromabwärts der Säule durchgeführt wird.

9. Verfahren nach einem der vorhergehenden Ansprüche, wobei Schritt c) durch Zugeben des Lösungsmittels in zwei bis zehn aufeinanderfolgenden Portionen durchgeführt wird.

10. Verfahren nach einem der vorhergehenden Ansprüche, wobei das Volumen des in Schritt c) verwendeten Lösungsmittels zwischen dem 10- und 25-Fachen des Ausgangsvolumens des Plasmas beträgt.

11. Verfahren nach einem der vorhergehenden Ansprüche, wobei die Messung in Schritt e) in einer elektrochemischen Zelle durchgeführt wird, die eine Arbeitselektrode, eine Referenzelektrode und eine Gegenelektrode umfasst.

12. Verfahren nach Anspruch 11, wobei eine Glaskohlenstoffelektrode oder eine Platinelektrode als Arbeitselektrode verwendet wird, ein Silberdraht als Referenzelektrode verwendet wird und eine Platinspirale als Gegenelektrode verwendet wird.

## Revendications

1. Méthode de détection de l'imatinib dans un échantillon de plasma sanguin, comprenant les étapes suivantes :

   a) charger l'échantillon de plasma sur une colonne d'extraction de liquide simplifiée supportée sur une phase solide ;
   b) charger la colonne avec une solution aqueuse basique ;
   c) éluer l'imatinib de la colonne d'extraction liquide simplifiée supportée sur une phase solide à l'aide d'un solvant organique choisi parmi le tétrahydrofurane, l'éther méthyl-terbutylique, l'éther éthylique, l'acétonitrile, l'acétate d'éthyle, l'acétate de butyle et leurs mélanges, en recueillant en bas de la colonne une solution contenant de l'imatinib ;
   d) ajouter à la solution d'imatinib obtenue à l'étape c) de l'hexafluorophosphate de tétrabutylammonium à une concentration comprise entre 1 et 100 mM et ajouter à la même solution une solution concentrée d'acide phosphorique, d'acide acétique glacial et d'eau ultrapure ;
   e) analyser le mélange obtenu à l'étape d) par voltampérométrie inverse d'adsorption, dont la forme d'onde (potentiel, E, en fonction du temps, t) est constituée par une première période d'accumulation dans laquelle le potentiel est maintenu constant à une première valeur, une deuxième période de décroissance linéaire du potentiel jusqu'à une deuxième valeur, et une troisième période d'augmentation linéaire du potentiel jusqu'à revenir à la première valeur, dans laquelle lesdites première et deuxième valeurs de potentiel sont respectivement +1,1 V et +0,3 V par rapport à une électrode de référence.

2. Procédé selon la revendication 1, dans lequel à l'étape a) un volume de plasma compris entre 20 et 200 $\mu$l est utilisé.

3. Procédé selon l'une des revendications 1 ou 2, dans lequel l'étape a) est réalisée en utilisant un flux de gaz inerte ou en appliquant un vide en aval de ladite colonne.

4. Procédé selon l'une quelconque des revendications précédentes, dans lequel la solution basique utilisée à l'étape b) est une solution aqueuse d'une base choisie parmi l'hydroxyde de sodium, l'hydroxyde de potassium, le phosphate de potassium, le phosphate de sodium, le carbonate de sodium, le carbonate de potassium, le borate de sodium et l'ammoniac.

5. Procédé selon l'une quelconque des revendications précédentes, dans lequel, à l'étape b), une solution aqueuse d'ammoniac est utilisée et le pH de l'échantillon est porté à une valeur d'environ 10,5.

6. Procédé selon l'une quelconque des revendications précédentes, dans lequel l'étape b) est réalisée en utilisant un flux de gaz inerte ou en appliquant un vide en aval de ladite colonne.

7. Procédé selon l'une quelconque des revendications précédentes, dans lequel le solvant utilisé comme éluant à l'étape c) est l'acétate d'éthyle.

8. Procédé selon l'une quelconque des revendications précédentes, dans lequel l'étape c) est réalisée en utilisant un flux de gaz inerte ou en appliquant un vide en aval de ladite colonne.

9. Procédé selon l'une quelconque des revendications précédentes, dans lequel l'étape c) est réalisée en ajoutant ledit solvant en deux à dix portions consécutives.

10. Procédé selon l'une quelconque des revendications précédentes, dans lequel le volume de solvant utilisé dans l'étape c) est compris entre 10 et 25 fois le volume initial de plasma.

11. Procédé selon l'une quelconque des revendications précédentes, dans lequel la mesure de l'étape e) est réalisée dans une cellule électrochimique comprenant une électrode de travail, une électrode de référence et une contre-électrode.

12. Procédé selon la revendication 11, dans lequel une électrode de carbone vitreux ou une électrode de platine est utilisée comme électrode de travail, un fil d'argent est utilisé comme électrode de référence et une spirale de platine est utilisée comme contre-électrode.

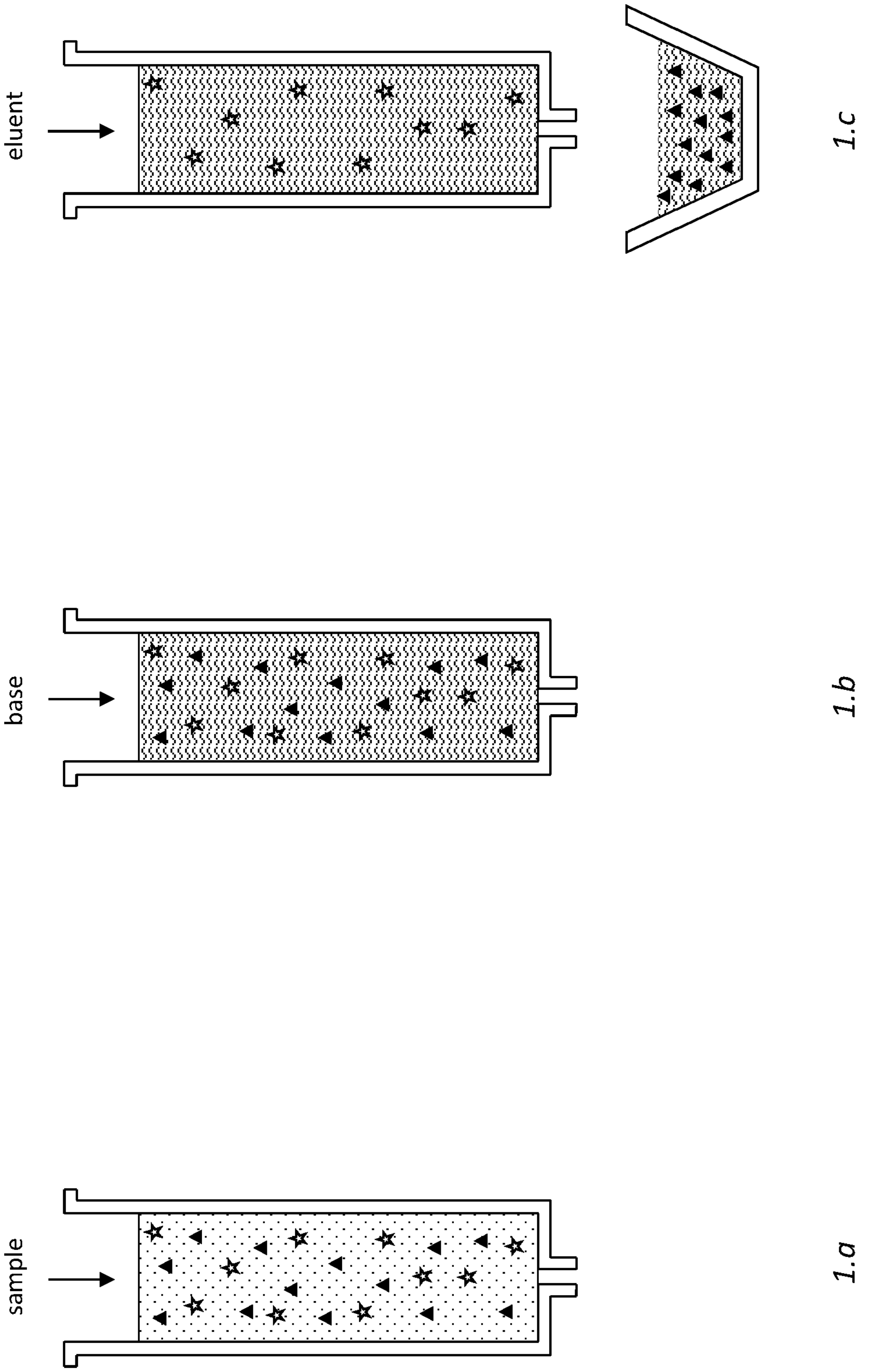


Fig. 1

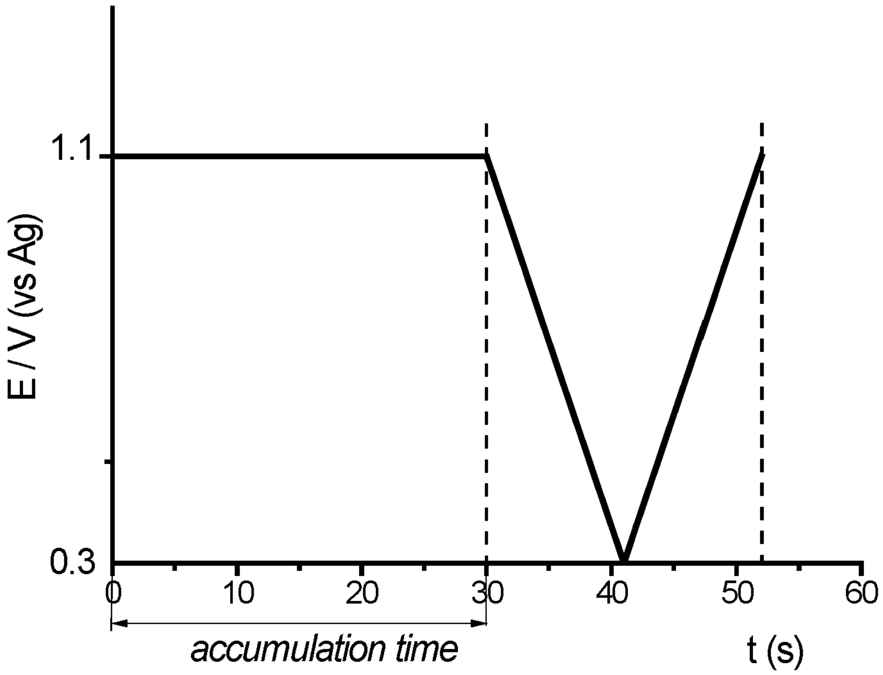
1.1
0.3
E / V (vs Ag)
0
10
20
30
40
50
60
accumulation time
t (s)

*Fig. 2*

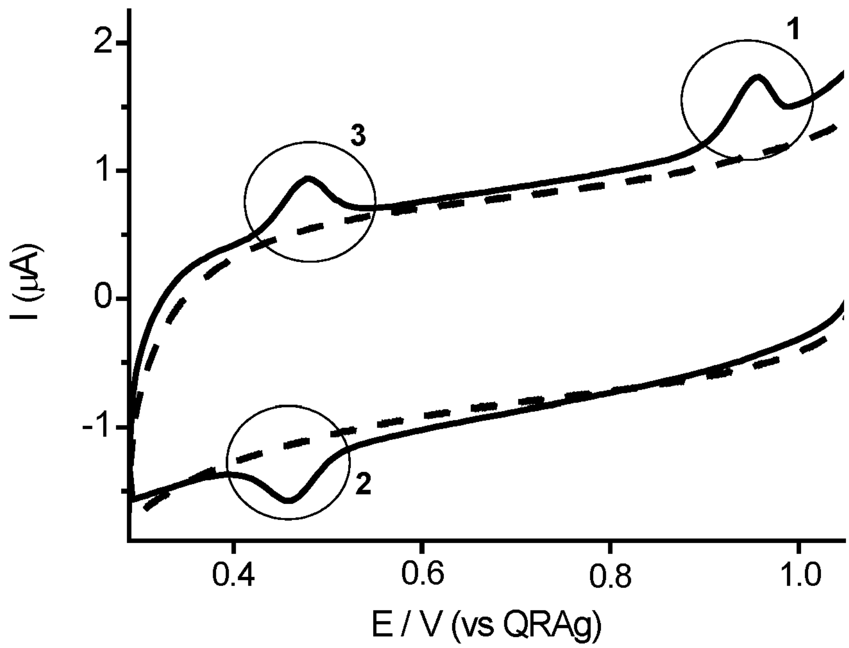
2
1
0
-1
I (μA)
1
2
3
0.4
0.6
0.8
1.0
E / V (vs QRAg)

*Fig. 3*

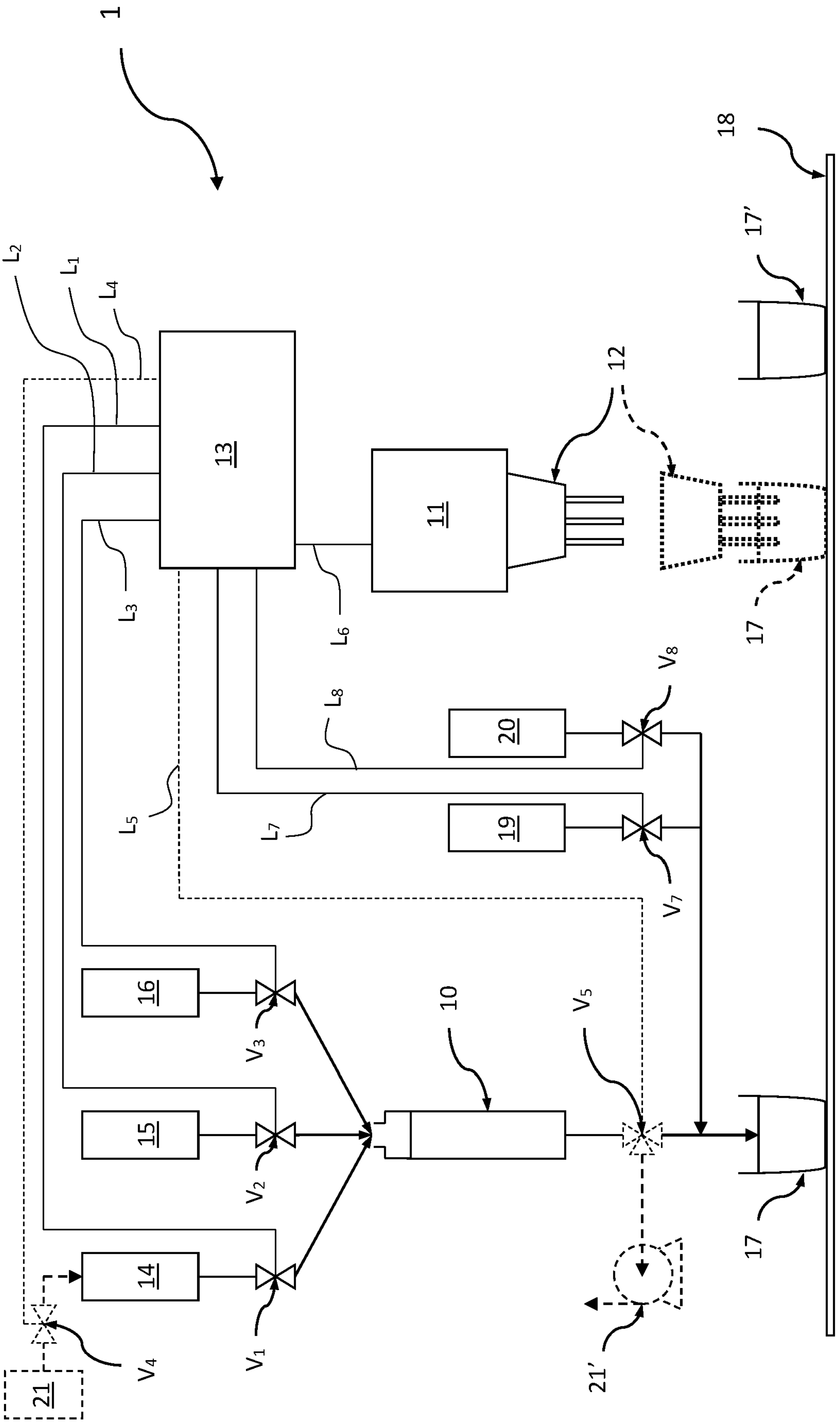
1
L2
L1
L4
13
L3
L6
11
12
18
17'
17
L8
L7
L5
20
19
V8
V7
16
15
14
10
V5
V3
V2
V1
V4
21
21'

Fig. 4

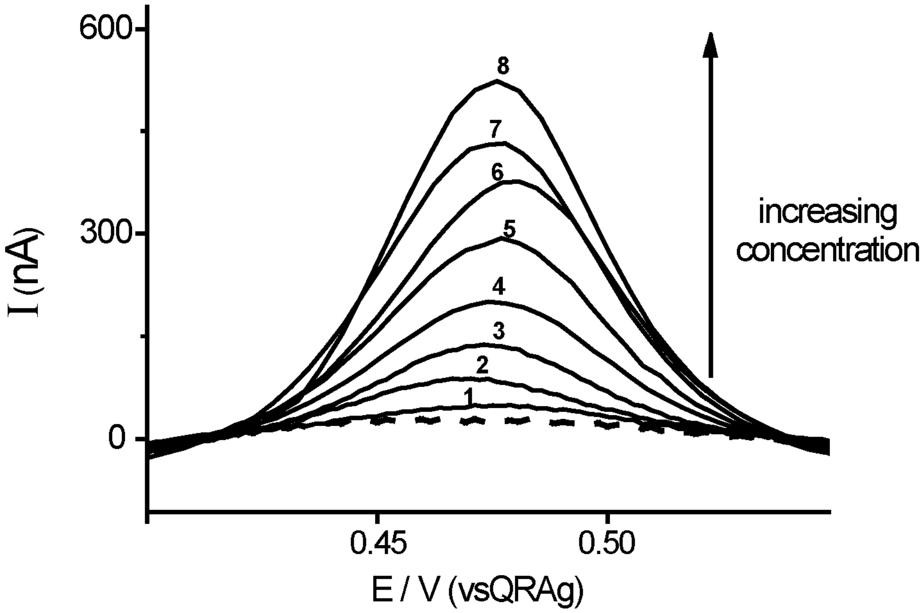
600
300
0
I (nA)
8
7
6
5
4
3
2
1
increasing
concentration
0.45
0.50
E / V (vsQRAg)

*Fig. 5*

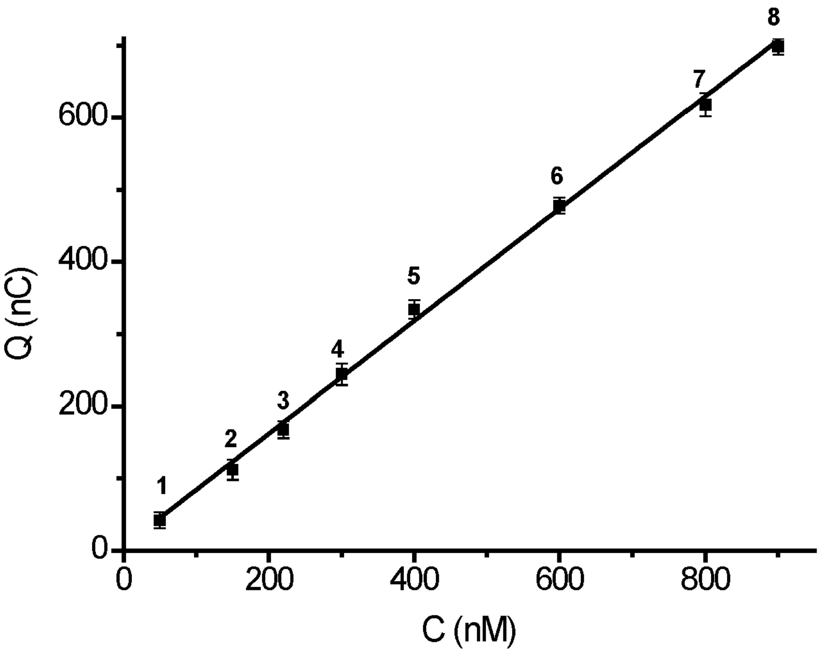
600
400
200
0
Q (nC)
1
2
3
4
5
6
7
8
0
200
400
600
800
C (nM)

*Fig. 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104804079 A
- EP 2251042 A2
- CN 104483407 A


### Non-patent literature cited in the description


- **HAMMAM E. et al.** Voltammetric behavior and quantification of the anti-leukemia drug imatinib in bulk form, pharmaceutical formulation, and human serum at a mercury electrode. *Canadian Journal of Chemistry,* February 2011, vol. 82 (7), 1203-1209
- **RODRIGUEZ J. et al.** Voltammetric determination of Imatinib (Gleevec) and its main metabolite using square-wave and adsorptive stripping square-wave techniques in urine samples. *Talanta,* April 2005, vol. 66 (1), 202-9
- **RODRIGUEZ et al.** Electrochemical sensor for leukemia drug imatinib determination in urine by adsorptive striping square wave voltammetry using modified screen-printed electrodes. *Electrochimica Acta,* March 2018, vol. 269 (4